# EUROPEAN PATENT SPECIFICATION

(11) **EP 1 551 464 B1**
(45) Date of publication and mention of the grant of the patent: **01.08.2007**
(21) Application number: 03794388.3
(22) Date of filing: 02.09.2003
(51) Int. Cl.: A61L 2/16

(54) **USE OF PARABEN COMPRISING COMPOSITIONS**
VERWENDUNG VON PARABEN-VERBINDUNGEN ENTHALTENDEN ZUSAMMENSETZUNGEN
UTILISATION DE COMPOSITIONS COMPRENANT DES PARABÈNES

(30) Priority: 05.09.2002 SE 0202625; 05.09.2002 US 407938 P
(43) Date of publication of application: 13.07.2005
(73) Proprietor: Mitra Medical AB, 223 70 Lund (SE)
(72) Inventor: SANDBERG, Bengt, E., B., S-245 62 Hjärup (SE); NILSSON, Rune, S-226 48 Lund (SE); RUDBÄCK, Charlotte, S-237 37 Bjärred (SE)
(74) Representative: Asketorp, Göran
(86) International application number: PCT/SE2003/001356
(87) International publication number: WO 2004/022111

(56) References cited:
- US-A- 4 619 935
- US-A- 5 079 236
- DATABASE WPI Week 199315, Derwent Publications Ltd., London, GB; Class A96, AN 1993-120433, XP002986690 & JP 5 057 015 A (ASAHI MEDICAL CO LTD) 09 March 1993

## Description

The present invention relates to the use of an aqueous antimicrobial preservation composition for eliminating or reducing the microbial content of a microbially contaminated separation matrix as well as a method of producing such a separation matrix with eliminated or reduced microbial content.

Separation matrices are nowadays included in many laboratory and industrial processes. These systems can be more or less specific for the components to be adsorbed. Such components may constitute the desired product or may constitute impurities or other undesired substances to be separated from the desired component by adsorption to the matrix. A common feature of many separation systems is separation matrices based on polymeric materials that are compatible with water and form their active configuration in contact with an aqueous solution (xerogels). This group of matrices includes, but is not limited to, polyacrylamide, celluloses, agaroses, and other polysaccharides.

However,in the presence of water these matrices may be susceptible to microbial growth. Microbial growth is a particular problem in these cases when a sterilization of the matrix by means of autoclaving, chemical sterilisation or irradiation is not applicable. This could be due to intrinsic sensitivity of the matrix or that a ligand linked to the matrix, covalently or non-covalently, is inactivated or altered by the sterilisation procedure. An example is when the ligand comprises recognition moieties that are based on protein or peptide structures. However, a number of other non-proteineous structures are also known to be sensitive to common sterilization techniques.

There are a number of applications where the prevention of microbial growth is of particular importance. One such application is the purification of protein-based pharmaceuticals or in vivo diagnostic products. Particularly, in the case where neither the separation matrix nor the final product can be properly sterilised according to any of the standard methods used for sterilisation. One example of this is when proteins immobilized to a matrix are utilized in the separation process, such as immobilized Protein A for the purification of immunoglobulins, or immobilized immunoglobulins for the purification of various substances interacting with the same. Yet another example is the retrieval of biotinylated substances by the use of separation matrices coated with avidin or streptavidin.

Even in cases where the final product can be subjected to an end-product sterilisation, it is of paramount importance to avoid any uncontrolled growth of microorganisms during the processes and storage prior to the end-product sterilisation. The problem of microbial contamination is not restricted to the presence of viable organisms but include toxins, pyrogens or other harmful products or metabolites released by such organisms. An example of such systems is the various types of endotoxins.

Yet another application of separation matrices requiring the prevention of microbial growth is the purification of nucleic acid materials. Here, even minute amounts of DNA material originating from contaminating microbial growth may interfere with analytical methods, gene splicing, or the cloning of genetically designed material, particularly if the latter is used in the production of pharmaceutical products or medical devices. Hence, there are numerous applications where the prevention of microbial growth in separation matrices is crucial. Similar problems may also arise in the separation of proteins, peptides or specific carbohydrate molecules due to microbial release of impurities of similar nature.

Consequently, there is a need for an efficient and adequate treatment of most separation matrices, whereby their contaminating microorganisms can be reduced or totally eliminated. This is of paramount importance if the separation matrix is intended to be used in a housing of a device where harmful substances are removed from blood or other body fluids being passed through the device, which is applied intra, para, or extracorporally.

Clinical extracorporeal adsorption systems for processing human plasma are available for the removal of immunoglobulin by the use of Protein-A in the treatment of autoimmune diseases (Bygren et al., Lancet 1985, 1295-1296) or to avoid early rejection of transplanted organs (Palmer et al., Lancet 1989, 10-12), and for the removal of low-density lipoprotein (LDL), in the treatment of hyper-cholesterolemia by the use of immobilized anti-LDL antibodies (duMoulin et al., 1990 in Treatment of Severe Hyper-cholesterolemia in the Prevention of Coronary Heart Disease, pp 170-174).

Extracorporeal devices have been described, which utilizeimmobilized anti-species antibodies, methods for, and the use of such devices for the removal of therapeutic or imaging antibodies in connection with immunotargeting of tumours (Henry CA, 1991, Vol.18, pp.565; Hofheinz D et al, Proc. Am. Assoc. Cancer Res. 1987 Vol. 28, pp. 391; Lear JL, et al., Radiology 1991, Vol.179, pp.509-512; Johnson TK, et al., Antibody Immunoconj. Radiopharm. 1991, Vol. 4, pp.509; Dienhart DG, et al., Antibody Immunoconj. Radiopharm. 1991, Vol. 7, pp.225; DeNardo GL, et al., J. Nucl. Med. 1993, Vol. 34, pp. 1020-1027; DeNardo GL, et al., J. Nucl. Med. 1992b, Vol. 33, pp. 863-864; DeNardo S.J., et. al. J. Nucl. Med. 1992a, Vol. 33, pp. 862-863; U.S. 5,474,772; Australian patent no. 638061, and European patent application no. 90914303.4).

In order to make the blood clearance more efficient and to enable the processing of whole blood, rather than blood plasma, the medical agent (e.g. the tumour specific monoclonal antibody carrying cell killing agents or radionuclides for tumour localization) has been biotinylated and cleared by means of an affinity (e.g. biotin-binding) column. A number of publications provide data which prove this technique to be both efficient and practical for the clearance of biotinylated and radionuclide labelled tumour specific antibodies (Norrgren K, et al., Antibody Immunoconj. Radiopharm. 1991, Vol. 4, pp. 54; Norrgren K, et. al., J. Nucl. Med. 1993, Vol. 34, pp. 448-454; Garkavij M, et. al. Acta Oncologica 1996, Vol. 53, pp.309-312; Garkavij M, et. al., J. Nucl. Med. 1997, Vol. 38, pp. 895-901). The European patent application no. 92903 020.3 describes these processing applications.

When used in connection with an extracorporeal circulation treatment a complete sterility of the separation matrix as well as the inside of the housing in contact with the same must be guaranteed. It should also be possible to sterilize the matrices of extracorporeal devices before use. In addition, it should be possible to prepare the device by aseptic processing under good-manufacturing- practice conditions.

Furthermore, in order to avoid unwanted effects on contents of aqueous solution to be treated, the method of choice - either wet heat, radiation, or chemical treatment - must not cause any chemical reaction with the matrix material and/or the housing. Such chemical reactions include chemical or structural changes, chain scission, cross-linking or a significant alteration in mechanical properties in the materials used in extracorporeal devices.

In the case of sterilization with ethylene oxide or formic aldehyde, such parameters as solubility, adsorption and desorption of the sterilization agent are crucial, due to physical and chemical sorption of the agent to the separation matrix as well as to surrounding plastic material. Owing to their high toxicity, a precise degassing time following sterilization is usually required.

Today, most currently available extracorporeal devices utilize supports made of agarose, silica, polyacrylate, or polyvinylacohol, which are sterilized by means of steam or thiomersal (merthiolate). However, no efficient sterilization of a separation matrix can up to now be performed when its ligand is a protein and the matrix is in a wet stage or by other means surrounded by water.

In EP 179420 a process is shown for steam-sterilizing of a matrix for use in connection with extracorporeal circulation treatment, and in US 5,283,034 a method and composition is described, which permits sterilization of surfaces coupled with biologically active moieties by ionizing radiation.

Different preservation agents have also been used in extracorporeal systems, such as azide and thiomersal. However, the use of these substances has been questioned since they may adsorb to the different materials used and they are thus difficult to wash away.
In both these examples acute and long-term toxicity constitutes additional problems. In the case of thiomersal, the allergic hazard for the patient as well as for the health professionals is a reality.

The document DATABASE WPI Week 199315, Derwent Publications Ltd., London, GB; Class A96, AN 1993-120433, describes the sterilization of an adsorbing agent for blood cleaning with H2S2O7 and water vapor.

Other useful preservation agents are not soluble in water, and some of them interact over time either with the ligand or the plastic material used in the housing. For example, benzyl alcohol makes the plastic brittle. Such preservation agents are also difficult to wash away, a property, which is also coupled to the poor solubility.

Furthermore, the preservation composition must react neither with the separation matrix nor with the housing with the formation of unwanted products, for example toxic substances. It is also important that the preservation composition does not reduce the binding efficiencies of the ligands used for the separation.

Often the separation matrix and the housing are produced at different locations as well as stored and/or transported before the manufacture of the final extracorporeal device. Such a handling requires a long-term antimicrobial effect without the preservation composition affecting neither the separation matrix nor the container used for storage and transport. Furthermore, the antimicrobial preservation composition should be easy to wash away from the separation matrix in connection with the filling of the housing of the separation device. In this way the separation matrix can be kept sterile until it is aseptically packed in its final device.

An efficient sterilization or preservation is also important when of re-usable separation matrices are to be treated. This is-especially the case when such matrices forms a part of a re-usable extracorporeal device intended for the processing of blood, plasma or other body fluids.

The support or matrix used in extracorporeal devices is often a polymeric porous material in beaded form, which is to be filled into the housing, the beads having a size sufficient to provide the requisite space between the beads when packed into the same. The support can also be a microfiltration hollow-fiber or a flat sheet membrane in order to minimize pressure drops.

Most of these separation systems utilize a solution to be treated as well as a separation matrix comprising a proteineous or other organic material, which is susceptible to microbial growth. The solution to be treated can be sterilized, for example by means of filtration, or is supplied as a body liquid. However, the separation matrix as well as its surrounding liquid represent a problem since it is microbially contaminated if not properly handled.

A preservation composition for a separation matrix and its surrounding fluid must have a static or cidal effect on both bacteria and fungi. Furthermore, toxicology data on the components of the composition must be available. Preferably, it should previously have been used in a pharmaceutical preparation, and the requirements for such preservatives with respect USP as well as the European Pharmacopeia must be fulfilled.

Parabens are among those biocides which affect cell membranes by altering the membrane potential or electron transport. They are most active against yeasts and molds. The parabens are esters of p-hydroxybenzoic acid. The two most common esters are methyl and propyl parabens, which are approved for food use in USA under GRAS classification. The parabens have not been as widely used as antimicrobial agents for other applications. Applications primarily include the food industry and parental medicinal products.

Prickett et al. (J. Pharm. Sci., vol 50 (4), pp. 316-320, 1961) have studied the potentiation of parabens in liquid oral pharmaceutical formulations by low concentrations of propylene glycol. They found that the potentiating effect of propylene glycol on the antimicrobial activity of the parabens may be utilized to increase the preservative action in formulations difficult to protect against microbial spoilage and to reduce the concentrations of parabens, thereby improving the flavor and consumer acceptance of liquid oral preparations.

The purpose of the invention is to eliminate the drawbacks mentioned above and the invention has thus obtained the characterizing features of claim 1 and 11, respectively.

Accordingly, this invention describes the surprising findings that an aqueous composition comprising at least one alkyl paraben can be used for eliminating or reducing the microbial content of a matrix, which is to be used in a housing of a separation device. Such a composition can for example be used for separation matrices in chromatography and process industry applications for the separation of medical products. It is especially suitable for sterilizing and/or storing a separation matrix, which is to be used in a housing of a device for extracorporeal circulation treatment.

In a method of producing a separation matrix with eliminated or reduced microbial content, the method comprises the steps of
providing the separation matrix, microbially contaminated, in a housing or container;
adding an aqueous antimicrobial preservation composition, which comprises at least one alkyl paraben, to the separation matrix in the housing or container;
allowing the aqueous antimicrobial preservation composition to exert its effect in the housing or container until the number of colony forming units (CFU) per g preservative composition is sufficiently reduced; and
rinsing the aqueous antimicrobial preservation composition from the housing or container.

Preferably, the aqueous antimicrobial preservation composition is allowed to exert its effect until the number of CFU per g preservative composition fulfils US and/or European Pharmacopeia test protocol.

The aqueous antimicrobial preservation compositions can be produced under GMP and ISO-9002/EN46002. Preferably, the compositions are sterilized before they are added to the separation matrix. Suitable sterilization methods are for example steam and filter sterilization.

The separation matrix material can be any supporting material that is commonly used as an immobilization matrix. Preferably, the separation matrix comprises a polysaccharide gel such as agarose. In a preferred embodiment the immobilized ligand is a protein, such as but not limited to, Protein A or other immunoglobulin binding proteins or peptides, or immunoglobulins or haptens binding various types of substances. In the most preferred embodiment the immobilized ligand is a biotin binding protein, such as but not limited to, avidin or streptavidin and derivatives thereof.

Suitable parabens are methyl paraben, ethyl paraben, propyl paraben, and butyl paraben.

The total concentration of the parabens as well as their relative distribution is of importance for the antimicrobial effect. Preferably, the concentration of each alkyl paraben in the composition decreases with increasing alkyl number (i.e. increasing number of carbon atoms). A suitable concentration of methyl paraben is between 0.5 and 2 g•l⁻¹, a suitable concentration of ethyl paraben is between 0.01 and 0.5 g•l⁻¹, a suitable concentration of propyl paraben is between 0.25 and 0.25 g•l⁻¹, and a suitable concentration of butyl paraben is at least 0.002 g•l⁻¹.

However, alkyl parabens are hard to maintain in solution, due to their tendency to precipitate. Thus, it is an advantage if the aqueous antimicrobial preservation composition further comprises a solubility increasing agent at a concentration that is sufficient to maintain the at least one alkyl paraben in solution during long time storage and/or storage conditions at ambient temperature (+4 to +25°C).

The solubility-increasing agent can be a higher alcohol or a polyalcohol, such as glycerol, polyethylene glycol, or propylene glycol. Preferably, the solubility-increasing agent is propylene glycol.

When propylene glycol is used, it is sufficient that its concentration is not more than 20 g•l⁻¹.

It should be noted that propylene glycol will not only aid solubility, and thus the stability of the aqueous antimicrobial preservation composition, but it will also potentiate the antimicrobial activity of the parabens therein and thus result in a maximum preservative effect. The antimicrobial preservastion composition should be allowed to exert its effect for at least 6 h, whereby the microbial content of the separation matrix is eliminated or reduced independent on the application used.

### EXAMPLES

The invention will now be further described and illustrated by reference to the following examples. It should be noted, however, that these examples should not be construed as limiting the invention in any way.

### EXAMPLE 1. PRESERVATIVE STUDIES.

Antimicrobial preservation compositions were prepared in the storage solution shown below.

**Storage solution**

| | |
|---|---|
| Citric acid | 0.79 mM |
| Tri-Sodium citrate | 49.21 mM |
| Sodium chloride | 0.15 M |
| Water | |
| pH 7.0 ±0.1 | |

A pH of 7.0 is ideal for bio-compatibility.

The following preservative compositions were studied. The actual commercial products tested were obtained from Nipa Laboratories Lmt, UK, and are given as registered trade names within parenthesis.

| | |
|---|---|
| Pres. (1) : | a mixture of |
| | phenoxyethanol, |
| | methyl paraben, |
| | propyl paraben, and |
| | 2-bromo-2-nitropropane-1,3-diol; |
| | (0.3 % Nipaguard BPX^{®}) |
| | |
| Pres. (2) : | a mixture of |
| | methyl paraben, 0.83 g•l⁻¹, |
| | ethyl paraben, 0.17 g•l⁻¹, and |
| | propyl paraben, 0.12 g•l⁻¹; |
| | (0.1 % Nipasept^{®}) |
| | |
| Pres. (3) : | a mixture of |
| | methyl paraben, 1.25 g•l⁻¹, |
| | ethyl paraben, 0.25 g•l ⁻¹, and |
| | propyl paraben, 0.18 g•l⁻¹; |
| | (0.15 % Nipasept^{®}) |
| | |
| Pres. (4) : | phenoxyethanol, 5.0 g•l ⁻¹; |
| | (0.5 % Phenoxetol^{®}) |
| | |
| Pres. (5) : | a mixture of |
| | methyl paraben, 0.83 g•l⁻¹, |
| | ethyl paraben, 0.17 g•l⁻¹, |
| | propyl paraben, 0.12 g•l⁻¹, and |
| | phenoxyethanol, 5.0 g•l⁻¹; |
| | (0.1 % Nipasept^{®} + 0.5 % Phenoxetol^{®}) |
| | |
| Pres. (6) : | a mixture of |
| | methyl paraben, 0.80 g•l ⁻¹, and |
| | ethyl paraben, 0.02 g•l⁻¹; |
| | (0.08 % Nipagin M^{®} + 0.02 % Nipagin A^{®} |
| | |
| Pres. (7) : | a mixture of |
| | methyl paraben, 0.80 g•l⁻¹, |
| | ethyl paraben, 0.02 g•l⁻¹, and |
| | butyl paraben, 0.005 g•l⁻¹; |
| | (0.08 % Nipagin M^{®} + 0.02 % Nipagin A^{®} |
| | + 0.005 % Nipabutyl^{®}) |
| | |
| Pres. (8) : | a mixture of |
| | methyl paraben, 0.70 g•l⁻¹, |
| | ethyl paraben, 0.02 g•l⁻¹ and |
| | butyl paraben, 0.005 g•l⁻¹; |
| | (0.07 % Nipagin M^{®} + 0.02 % Nipagin A^{®} |
| | + 0.005 % Nipabutyl^{®}) |
| | |
| Pres. (9) : | a mixture of |
| | methyl paraben, 0.80 g•l⁻¹, |
| | ethyl paraben, 0.15 g•l⁻¹ and |
| | butyl paraben, 0.005 g•l⁻¹; |
| | (0.08 % Nipagin M^{®} + 0.15 % Nipagin A^{®} |
| | + 0.005 % Nipabutyl^{®}) |
| | |
| Pres. (10) : | a mixture of |
| | methyl paraben, 0.80 g•l⁻¹, |
| | ethyl paraben, 0.02 g•l⁻¹, and |
| | butyl paraben, 0.04 g•l⁻¹; |
| | (0.08 % Nipagin M^{®} + 0.02 % Nipagin A^{®} |
| | + 0.004 % Nipabutyl^{®} |

Pres. (1) was prepared by using the storage solution, pre-sterilised by autoclaving, sterile implements and aseptic techniques. The other preservative compositions were sterilised by autoclaving.

### Microbial Challenge Test

### EP 1997 - test protocol

Five 20 g portions of each preservative composition were transferred to sterile containers and inoculated separately with 0.2 ml culture of the test organisms detailed below. The inoculated sample portions were mixed by means of a vortex mixer and stored at room temperature. The challenge test protocol of the EP 1997 was then followed.

### USP 23 - test protocol

Five 20 g portions of each preservative composition were inoculated with 0.1 ml culture of the test organisms as detailed below by the initial column level in the number of colony forming units (CFU) per g preservative composition. The inoculated sample portions were mixed by means of a vortex mixer and stored at room temperature. The challenge test protocol of the USP was then followed.

| | | INITIAL INOCULUM LEVEL | |
|---|---|---|---|
| | | CFU per g | |
| TEST SPECIES | | EP 1997 | USP XXIII |
| *Pseudomonas aeruginosa* | NCIMB 8626 | 1.1x10⁶ | 5.6x10⁶ |
| *Escherichia coli* | NCIB 8545 | 2.0x10⁶ | 1.0x10⁶ |
| *Staphylococcus aureus* | NCTC 10788 | 4.3x10⁶ | 2.2x10⁶ |
| *Candida albicans* | NCPF 3179 | 8.4x10⁵ | 4.2x10⁵ |
| *Aspergillus niger* | IMI 149007 | 1.0x10⁵ | 5.0x10⁴ |

### Test Results

The number of colony forming units (CFU) per g preservative composition are given and compared with a storage solution only (unpreserved).

### EP 1997

**Pres. (1)** 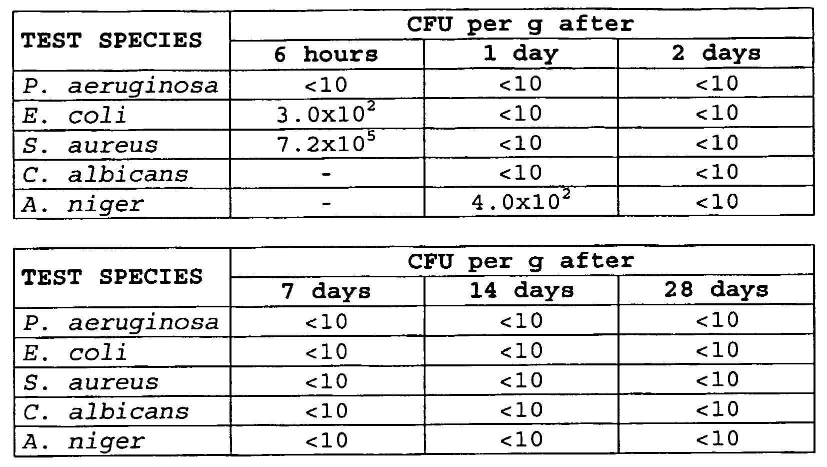
**Pres. (2)** 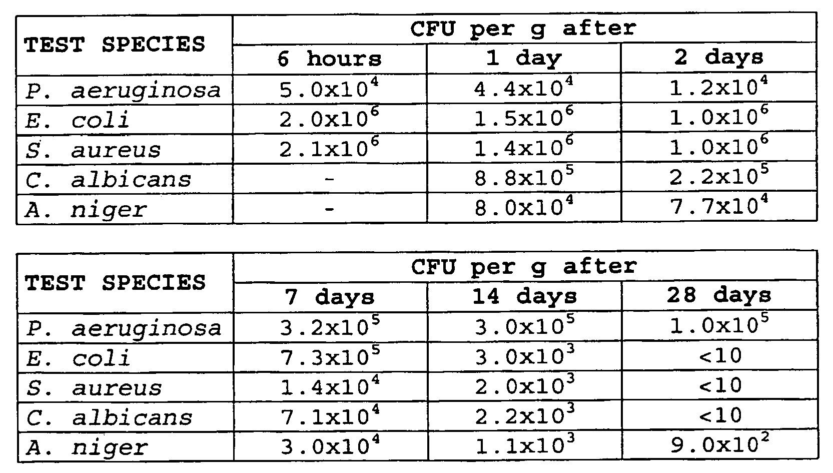
Pres. (3) 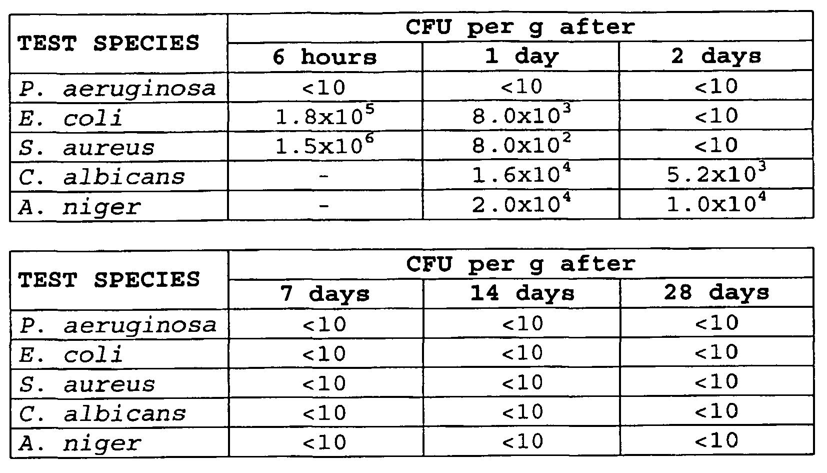
**Pres. (4)** 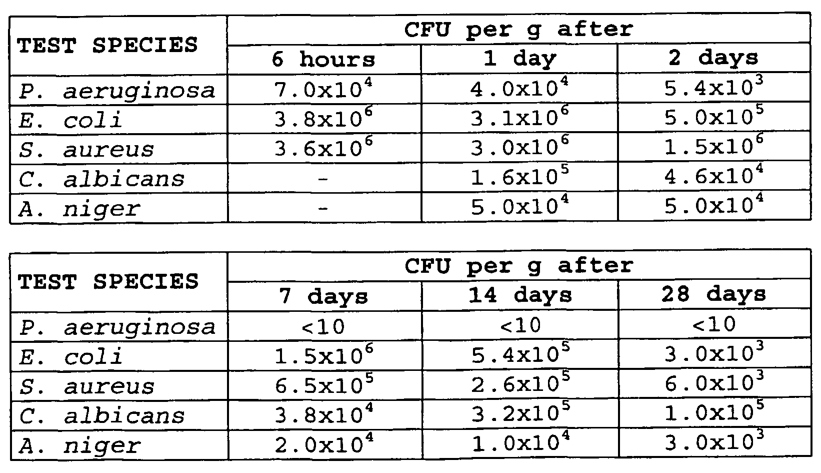
**Pres. (5)** 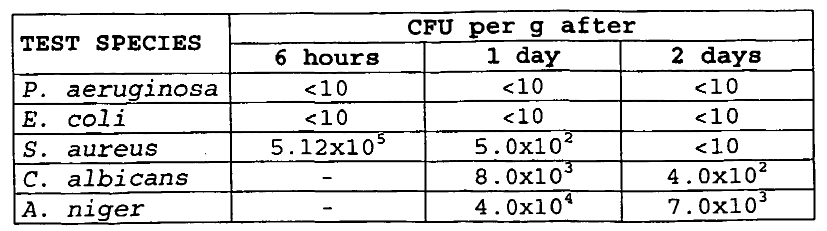 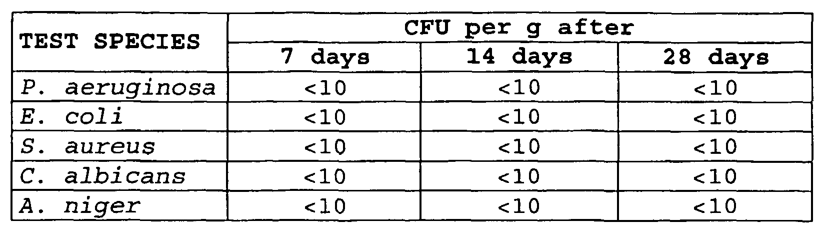
**Pres. (6)** 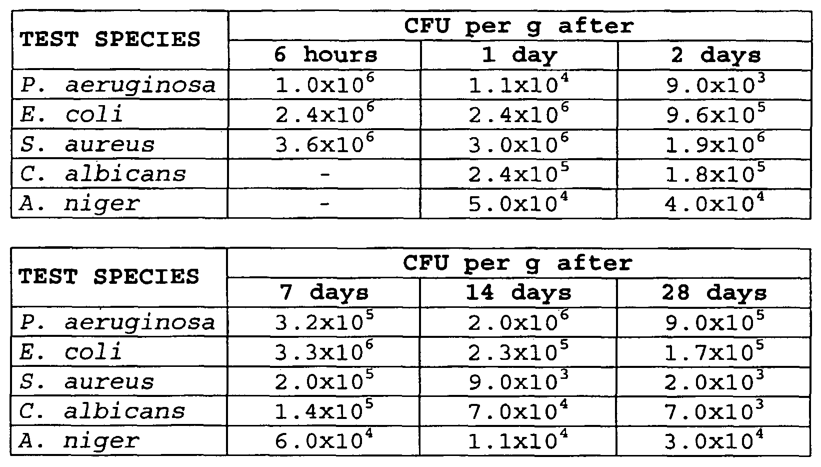
**Pres. (7)** 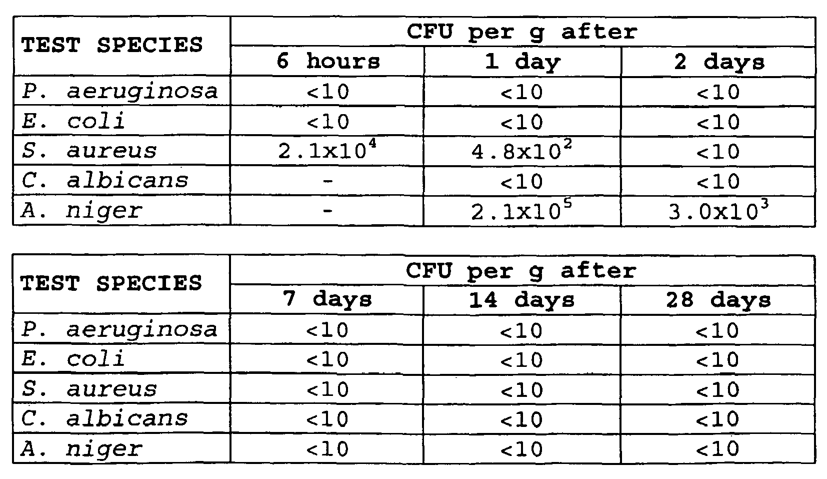
Unpreserved

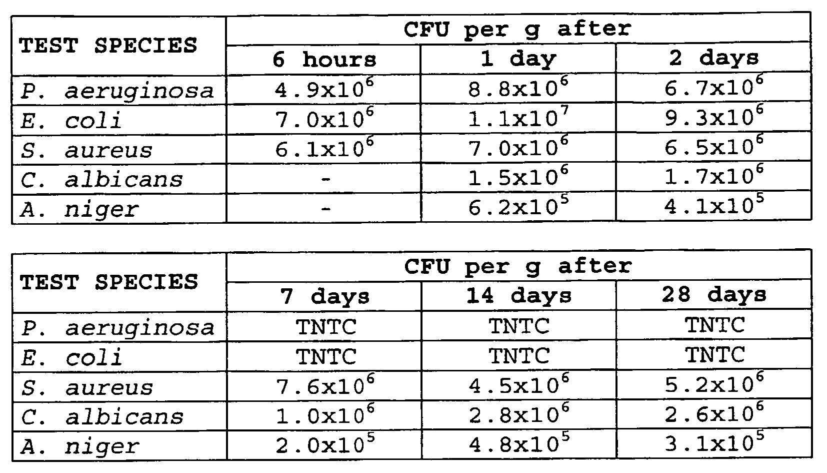

### USP 23

**Pres. (1)**

| **TEST SPECIES** | **CFU per g after:** | | | |
|---|---|---|---|---|
| | **7 days** | **14 days** | **21 days** | **28 days** |
| *P. aeruginosa* | <10 | <10 | <10 | <10 |
| *E. coli* | <10 | <10 | <10 | <10 |
| *S. aureus* | <10 | <10 | <10 | <10 |
| *C. albicans* | <10 | <10 | <10 | <10 |
| *A. niger* | <10 | <10 | <10 | <10 |

**Pres. (2)**

| **TEST SPECIES** | **CFU per g after:** | | | |
|---|---|---|---|---|
| | **7 days** | **14 days** | **21 days** | **28 days** |
| *P. aeruginosa* | 9.1x10³ | 5.2x10³ | 7.1x10³ | <10 |
| *E. coli* | 5.0x10⁵ | 9.2x10³ | 2.1x10³ | <10 |
| *S. aureus* | 7.1x10³ | 4.0x10² | <10 | <10 |
| *C. albicans* | 5.2x10⁴ | 3.0x10³ | <10 | <10 |
| *A. niger* | 1.0x10³ | <10 | <10 | <10 |

Pres. (3)

| **TEST SPECIES** | **CFU per g after:** | | | |
|---|---|---|---|---|
| | **7 days** | **14 days** | **21 days** | **28 days** |
| *P. aeruginosa* | <10 | <10 | <10 | <10 |
| *E. coli* | <10 | <10 | <10 | <10 |
| *S. aureus* | <10 | <10 | <10 | <10 |
| *C. albicans* | <10 | <10 | <10 | <10 |
| *A. niger* | <10 | <10 | <10 | <10 |

**Pres. (4)**

| **TEST SPECIES** | **CFU per g after:** | | | |
|---|---|---|---|---|
| | **7 days** | **14 days** | **21 days** | **28 days** |
| *P. aeruginosa* | <10 | <10 | <10 | <10 |
| *E. coli* | 6.0x10⁵ | 1.0x10⁵ | 8.0x10⁴ | 4.0x10² |
| *S. aureus* | 3.1x10⁵ | 7.1x10⁴ | 4.5x10⁴ | 4.9x10³ |
| *C. albicans* | 1.0x10⁴ | 9.0x10³ | 3.0x10³ | <10 |
| *A. niger* | 6.0x10³ | 4.0x10³ | 1.0x10³ | <10 |

**Pres. (5)**

| **TEST SPECIES** | **CFU per g after:** | | | |
|---|---|---|---|---|
| | **7 days** | **14 days** | **21 days** | **28 days** |
| *P. aeruginosa* | <10 | <10 | <10 | <10 |
| *E. coli* | <10 | <10 | <10 | <10 |
| *S. aureus* | <10 | <10 | <10 | <10 |
| *C. albicans* | <10 | <10 | <10 | <10 |
| *A. niger* | <10 | <10 | <10 | <10 |

**Pres. (6)**

| **TEST SPECIES** | | | | |
|---|---|---|---|---|
| | **7 days** | **14 days** | **21 days** | **28 days** |
| *P. aeruginosa* | 8.2x10⁴ | 6.0x10⁴ | 3.0x10⁴ | 7.0x10³ |
| *E. coli* | 5.1x10⁴ | 3.3x10⁴ | 6.0x10⁴ | 1.2x10⁴ |
| *S. aureus C. albicans* | 9.0x10⁴ | 5.0x10⁴ | 4.1x10⁴ | 9.1x10³ |
| | 8.3x10⁴ | 6.3x10⁴ | 6.0x10⁴ | 2.9x10³ |
| *A. niger* | 4.0x10⁴ | 1.0x10⁴ | 7.0x10³ | 4.0x10³ |

Pres. (7)

| **TEST SPECIES** | **CFU per g after:** | | | |
|---|---|---|---|---|
| | **7 days** | **14 days** | **21 days** | **28 days** |
| *P. aeruginosa* | <10 | <10 | <10 | <10 |
| *E. coli* | <10 | <10 | <10 | <10 |
| *S. aureus* | <10 | <10 | <10 | <10 |
| *C. albicans* | <10 | <10 | <10 | <10 |
| *A. niger* | <10 | <10 | <10 | <10 |

**Unpreserved**

| **TEST SPECIES** | **CFU per g after:** | | | |
|---|---|---|---|---|
| | **7 days** | **14 days** | **21 days** | **28 days** |
| *P. aeruginosa* | 3.8x10⁶ | 4.7x10⁶ | 4. 2x10⁶ | 5.0x10⁶ |
| *E. coli* | 6.2x10⁶ | 9.0x10⁶ | 7.3x10⁶ | TNTC |
| *S. aureus* | 3.7x10⁶ | 4.3x10⁶ | 5.1x10⁶ | 8.2x10⁶ |
| *C. albicans* | 8.0x10⁵ | 1.2x10⁶ | 2.0x10⁶ | 3.7x10⁶ |
| *A. niger* | 8.0x10⁴ | 9.0x10⁴ | 1.0x10⁵ | 2.1x10⁵ |

### Conclusions

The EP 1997, A criteria (target), requires the bacteria to be reduced by at least log 2 at 6 hours, log 3 at 24 hours with no organisms recovered at 7 days and thereafter and yeasts/moulds reduced by at least log 2 at 7 days with no increase thereafter. The B criteria (minimum criteria) requires the bacteria to be reduced by at least log 1 at 24 hour, log 3 at 7 days with no increase at 14 days and thereafter and the yeast/mould be reduced by at least log 1 at 14 days with no increase thereafter.

The USP 23 requires the bacteria reduced by at least log 3 at 14 days with no increase thereafter. The yeasts/moulds should show no increase at 14 days and thereafter.

### SUMMARY - applying the above criteria

| **PRESERVATIVE SYSTEM** | **EP 1997** | | **USP** |
|---|---|---|---|
| | **Criteria A (TARGET)** | **Criteria B (minimum)** | |
| Pres. (1) | Fail | Pass | Pass |
| Pres. (2) | Fail | Fail | |
| Pres. (3) | Fail | Pass | Fail |
| Pres. (4) | Fail | Fail | Fail |
| Pres. (5) | Fail | Pass | Pass |
| Pres. (6) | Fail | Fail | Fail |
| Pres. (7) | Pass | Pass | Pass |
| Unpreserved | Fail | Fail | Fail |

Test results indicate that a combination of methyl paraben, 0.80 g•l ⁻¹, ethyl paraben, 0.02 g•l ⁻¹, and butyl paraben, 0.005 g•l⁻¹ (Pres (7)) should effectively preserve the storage solution to the standards of the EP 1997 (A target criteria), for parental preparations, and the USP 23.
The EP 1997 (B minimum criteria) is less stringent, as shown by the results obtained with Pres. (3) as well as Pres. (5).

Likewise, the USP 23 pass criteria is still less stringent than the EP 1997 (parentals), which is reflected by the effective results obtained with Pres. (1), Pres. (2), Pres. (3), Pres. (5) and Pres. (7).

### EXAMPLE 2. STABILITY STUDIES.

Storage solutions of antimicrobial preservation compositions will be stored for a shelf life of up to 1.5 years at 2-8°C. The preservative compositions tested in the microbial challenge test were stored at 4°C and -15°C for 14 days in order to determine the preservative stability.

The stability was tested visually after storing and eventual thawing from -15°C.

| **PRESERVATIVE SYSTEM** | **Stability after 14 days at:** | |
|---|---|---|
| | **4°C** | **-15°C** |
| Pres. (1) | Stable | Stable |
| Pres. (2) | Stable | Stable |
| Pres. (3) | Stable | Stable |
| Pres. (4) | Stable | Stable |
| Pres. (5) | Stable | Stable |
| Pres. (6) | Stable | Stable |
| Pres. (7) | Precipitate | Precipitate |

The combination of methyl paraben, 0.80 g•l^{- 1}, ethyl paraben, 0.02 g•l⁻¹ and butyl paraben, 0.005 g•l⁻¹ (Pres. (7)) was effective in microbial challenge tests but not stable at 4°C and -15°C as shown by the sedimentation of white sedimentation/crystals.

The stability of the following preservative systems were then determined.

| **PRESERVATIVE SYSTEM** | **Stability after 14 days at:** | |
|---|---|---|
| | **4°C** | **-15°C** |
| Pres. (8) | Stable | Stable |
| Pres. (9) | Stable | Stable |
| Pres. (10) | Stable | Stable |

### EXAMPLE 3. EFFECT OF INCREASING SOLUBILITY.

In order to reach Ph. Eur. criteria A for microbial effect while at the same time obtaining a sufficient solubility, a solubility increasing agent was added to the parabens of choice. Any substance compatible with water can be used, provided it is approved for medical use.
Preferably, propylene glycol is used to increase the solubility.

Thus, an evaluation of low temperature stability was performed with buffer solutions of methyl, ethyl, and propyl parabens of different concentrations and 2 or 5 % propylene glycol.

The following antimicrobial preservation compositions were prepared in the storage solution shown above, except that pH 6.0 was used instead of pH 7.0. By choosing pH 6.0, it is possible to use sodium salts of parabens, which cannot be used in acid solutions.

| | |
|---|---|
| Pres. (11) : | a mixture of |
| | methyl paraben, 0.83 g•l⁻¹, |
| | ethyl paraben, 0.17 g•l⁻¹, and |
| | propyl paraben, 0.12 g•l⁻¹; |
| | (0.10 % Nipasept^{®}) |
| | |
| Pres. (12) : | a mixture of |
| | methyl paraben, 1.00 g•l⁻¹, |
| | ethyl paraben, 0.20 g•l ⁻¹, and |
| | propyl paraben, 0.14 g•l ⁻¹; |
| | (0.12 % Nipasept^{®}) |
| | |
| Pres. (13) : | a mixture of |
| | methyl paraben, 1.16 g•l⁻¹, |
| | ethyl paraben, 0.24 g•l⁻¹, and |
| | propyl paraben, 0.17 g•l⁻¹; |
| | (0.14 % Nipasept^{®}) |
| | |
| Pres. (14) : | a mixture of |
| | methyl paraben, 1.33 g•l⁻¹, |
| | ethyl paraben, 0.27 g•l⁻¹, and |
| | propyl paraben, 0.19 g•l ⁻¹_{;} |
| | (0.16 % Nipasept^{®}) |
| | |
| Pres. (15) : | a mixture of |
| | methyl paraben, 1.49 g•l⁻¹, |
| | ethyl paraben, 0.31 g•l⁻¹, and |
| | propyl paraben, 0.22 g•l⁻¹; |
| | (0.18 % Nipasept^{®}) |
| | |
| Pres. (16) : | a mixture of |
| | methyl paraben, 1.66g•l⁻¹; |
| | ethyl paraben, 0.34 g•l⁻¹, and |
| | propyl paraben, 0.24 g•l⁻¹; |
| | (0.20 % Nipasept^{®}) |
| | |
| Pres. (17) : | a mixture of |
| | methyl paraben, 0.83 g•l⁻¹, |
| | ethyl paraben, 0.17 g•l⁻¹, |
| | propyl paraben, 0.12 g•l⁻¹, and |
| | propylene glycol, 20 g•l⁻¹; |
| | (0.10 % Nipasept^{®} + 2 % propylene glycol) |
| | |
| Pres. (18) : | a mixture of |
| | methyl paraben, 1.00 g•l⁻¹, |
| | ethyl paraben, 0.20 g•l⁻¹, |
| | propyl paraben, 0.14 g•l⁻¹, and |
| | propylene glycol, 20 g•l⁻¹; |
| | (0.12 % Nipasept^{®} + 2 % propylene glycol) |
| | |
| Pres. (19) : | a mixture of |
| | methyl paraben, 1.16 g•l⁻¹, |
| | ethyl paraben, 0.24 g•l⁻¹, |
| | propyl paraben, 0.17 g•l⁻¹, and |
| | propylene glycol, 20 g•l⁻¹; |
| | (0.14 % Nipasept^{®}) |
| | |
| Pres. (20) : | a mixture of |
| | methyl paraben, 1.33 g•l⁻¹, |
| | ethyl paraben, 0.27 g•l⁻¹, |
| | propyl paraben, 0.19 g•l⁻¹, and |
| | propylene glycol, 20 g•l⁻¹; |
| | (0.16 % Nipasept^{®} + 2 % propylene glycol) |
| | |
| Pres. (21) : | a mixture of |
| | methyl paraben, 1.49 g•l⁻¹, |
| | ethyl paraben, 0.31 g•l⁻¹, |
| | propyl paraben, 0.22 g•l⁻¹, and |
| | propylene glycol, 20 g•l⁻¹; |
| | (0.18 % Nipasept^{®}) |
| | |
| Pres. (22) : | a mixture of |
| | methyl paraben, 1.66g•l⁻¹, |
| | ethyl paraben, 0.34 g•l⁻¹, |
| | propyl paraben, 0.24 g•l⁻¹, and |
| | propylene glycol, 20 g•l⁻¹; |
| | (0.20 % Nipasept^{®} + 2 % propylene glycol) |
| | |
| Pres. (23) : | a mixture of |
| | methyl paraben, 0.83 g•l^{-1,} |
| | ethyl paraben, 0.17 g•l⁻¹, and |
| | propyl paraben, 0.12 g•l⁻¹; |
| | propylene glycol, 50 g•l^{-1;} |
| | (0.10 % Nipasept^{®} + 5 % propylene glycol) |
| | |
| Pres. (24) : | a mixture of |
| | methyl paraben, 1.00 g•l⁻¹, |
| | ethyl paraben, 0.20 g•l⁻¹, |
| | propyl paraben, 0.14 g•l⁻¹, and |
| | propylene glycol, 50 g•l⁻¹; |
| | (0.12 % Nipasept^{®} + 5 % propylene glycol) |
| | |
| Pres. (25) : | a mixture of |
| | methyl paraben, 1.16 g•l⁻¹, |
| | ethyl paraben, 0.24 g•l⁻¹, |
| | propyl paraben, 0.17 g•l⁻¹, and |
| | (0.14 % Nipasept^{®}) |
| | |
| Pres. (26) : | a mixture of |
| | methyl paraben, 1.33 g•l⁻¹, |
| | ethyl paraben, 0.27 g•l⁻¹, |
| | propyl paraben, 0.19 g•l⁻¹, and |
| | propylene glycol, 50 g•l⁻¹; |
| | (0.16 % Nipasept^{®} + 5 % propylene glycol) |
| | |
| Pres. (27) : | a mixture of |
| | methyl paraben, 1.49 g•l⁻¹, |
| | ethyl paraben, 0.31 g•l⁻¹, |
| | propyl paraben, 0.22 g•l⁻¹, and |
| | propylene glycol, 50 g•l⁻¹_{;} |
| | (0.18 % Nipasept^{®} + 5 % propylene glycol) |
| | |
| Pres. (28) : | a mixture of |
| | methyl paraben, 1.66g•l⁻¹, |
| | ethyl paraben, 0.34 g•l⁻¹, |
| | propyl paraben, 0.24 g•l ⁻¹, and |
| | propylene glycol, 50 g•l⁻¹; |
| | (0.20 % Nipasept^{®} + 5 % propylene glycol) |
| | |
| Pres. (29) : | a mixture of |
| | methyl paraben, 0.80 g•l⁻¹, |
| | ethyl paraben, 0.02 g•l⁻¹, and |
| | butyl paraben, 0.005 g•l⁻¹; |
| | (0.08 % Nipagin M^{®} + 0.02 % Nipagin A^{®} |
| | + 0.005 % Nipabutyl^{®}) |
| | |
| Pres. (30) : | a mixture of |
| | methyl paraben, 0.80 g•l⁻¹, |
| | ethyl paraben, 0.02 g•l⁻¹, |
| | butyl paraben, 0.005 g•l⁻¹, and |
| | propylene glycol, 20 g•l⁻¹; |
| | (0.08 % Nipagin M^{®} + 0.02 % Nipagin A^{®} |
| | + 0.005 % Nipabutyl^{®} + 2 % propylene glycol) |
| | |
| Pres. (31) : | a mixture of |
| | methyl paraben, 0.80 g•l⁻¹, |
| | ethyl paraben, 0.02 g•l⁻¹, |
| | butyl paraben, 0.005 g•l⁻¹, and |
| | propylene glycol, 50 g•l⁻¹; |
| | (0.08 % Nipagin M^{®} + 0.02 % Nipagin A^{®} |
| | + 0.005 % Nipabutyl^{®} + 5 % propylene glycol) |

These preservation compositions were sterilized by means of autoclaving, stored at 4°C for 14 days and then assessed visible for precipitation in the form of white sedimentation crystals.

### Test Reults

| | Precipitation |
|---|---|
| Pres. (11) | (+) |
| Pres. (12) | (+) |
| Pres. (13) | + |
| Pres. (14) | + |
| Pres. (15) | + |
| Pres. (16) | ++ |
| Pres. (17) | - |
| Pres. (18) | - |
| Pres. (19) | - |
| Pres. (20) | - |
| Pres. (21) | (+) |
| Pres. (22) | (+) |
| Pres. (23) | - |
| Pres. (24) | - |
| Pres. (25) | - |
| Pres. (26) | - |
| Pres. (27) | (+) |
| Pres. (28) | (+) |
| Pres. (29) | - |
| Pres. (30) | - |
| Pres. (31) | - |

The results show that the tested preservation composition Pres. (29) seems to be right at the edge of the solubility at the low storage temperature since none of the remaining tested preservation compositions were stable at the low storage temperature without the addition of the solubility increasing agent propylene glycol.

When the paraben concentrations were increased, a further addition of the solubility increasing agent did not improve the solubility (cf. Pres. (21) and Pres. (27)).

### Mirobial Challenge Test

The minimum effective preservative concentration was evaluated by further studying the preservation compositions Pres. (18), Pres. (19), Pres. (20), and Pres. (30).

Five 20 g portions of each solution were transferred to sterile containers and were inoculated separately with 0.2 ml culture of the test organisms detailed below. The inoculated sample portions were mixed by means of a vortex mixer and stored at room temperature. The challenge test protocol of the EP 1997 was then followed.

| Test organism | Initial inoculum level | |
|---|---|---|
| | CFU per g | |
| *Pseudomnas aeruginosa* | NCIMB 8626 | 5.3x10⁶ |
| *Escherichia coli* | NCIB 8545 | 1.2x10⁷ |
| *Staphylococcus aureus* | NCTC 10788 | 8.2x10⁶ |
| *Candida albicans* | NCPF 3179 | 1.3x10⁶ |
| *Asperaillus niger* | IMI 149007 | 5.8x10⁵ |

### Test Reults

### EP 1997

**Pres. (18)** 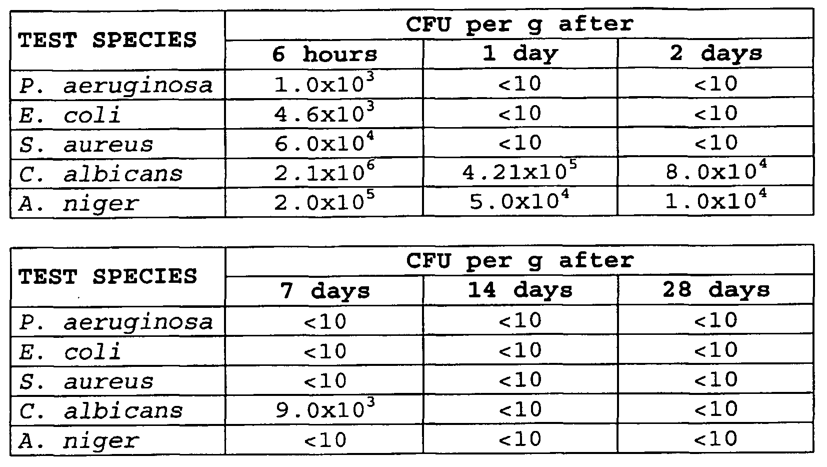
**Pres. (19)** 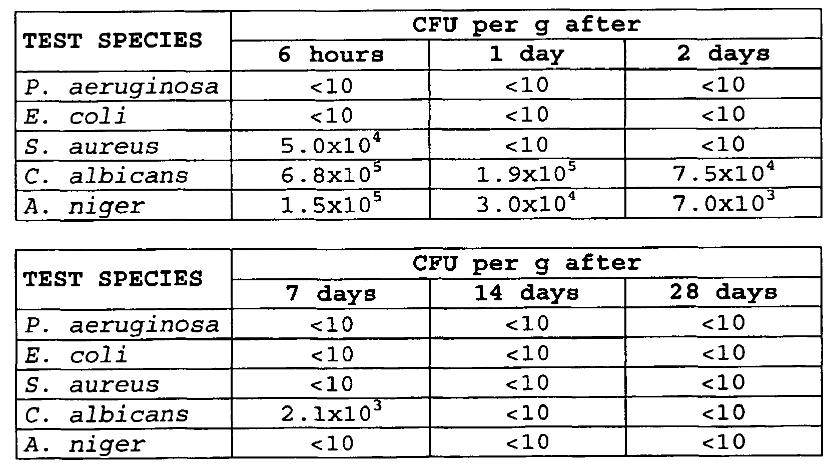
**Pres. (20)** 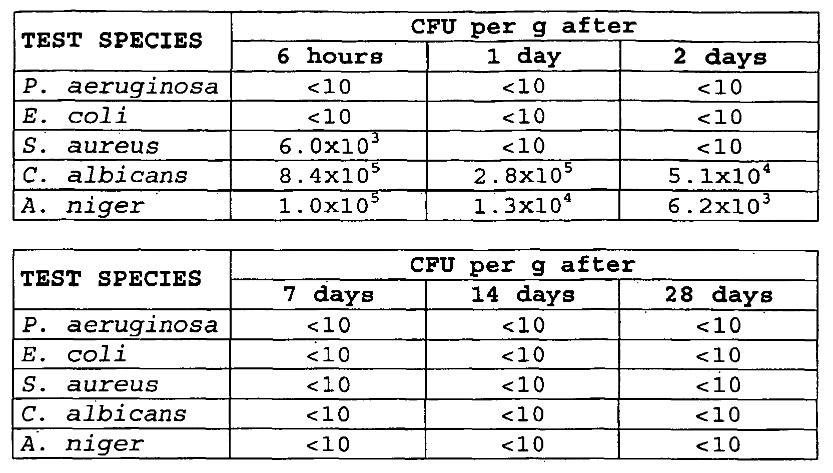
**Pres. (30)**

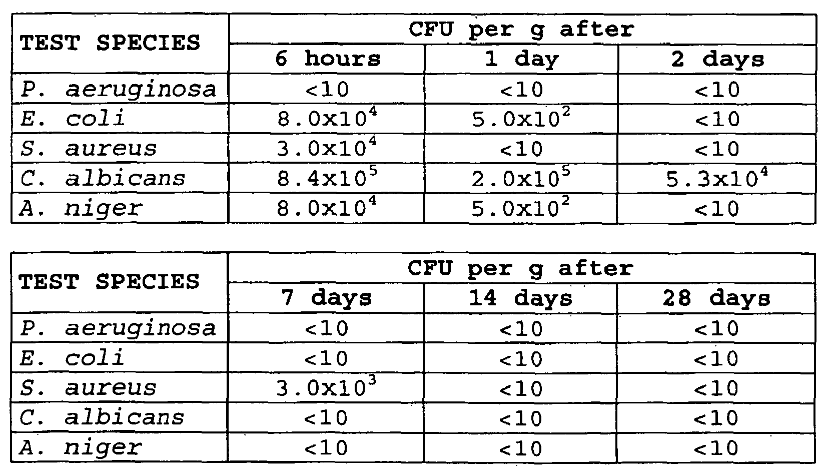

The EP 1997, A criteria (target), requires the bacteria to be reduced by at least log 2 at 6 hours, log 3 at 24 hours with no organisms recovered at 7 days and thereafter and yeasts/moulds reduced by at least log 2 at 7 days with no increase thereafter. The B criteria (minimum criteria) requires the bacteria to be reduced by at least log 24 1 hour, at log 3 at 7 days with no increase at 14 days and thereafter and the yeast/mould be reduced by at least log 1 at 14 days with no increase thereafter.

### SUMMARY - applying the above criteria

| **PRESERVATIVE SYSTEM** | **EP 1997** | |
|---|---|---|
| | **Criteria A (TARGET)** | **Criteria B (minimum)** |
| Pres. (18) | Pass | Pass |
| Pres. (19) | Pass | Pass |
| Pres. (20) | Pass | Pass |
| Pres. (30) | Pass | Pass |

The test results indicate that these antimicrobial preservation compositions should effectively preserve the storage solution to the standards of the EP 1997 A (target criteria).

The lowest total paraben concentration, at which precipitation at 4°C is obtained, is the antimicrobial composition Pres. (21). In order to obtain a safety margin in solubility of 50 % at 4°C the composition Pres. (18) is a preferred choice.

### EXAMPLE 4. SAFETY EVALUATION.

### Toxicology Data

A summary of toxicology data, abstracted from the scientific literature, is given below.

### Methyl paraben

| **TEST** | **RESULT** |
|---|---|
| Acute oral LD₅₀ (Mouse) | >8000 mg/kg |
| Intraperitoneal LD₅₀ (Mouse) | 960 mg/kg |
| Repeated oral dose (Rat) - 96 weeks | 900-1200 mg/kg/day gave no visible signs of toxicity or tissue changes in the major organs |
| Repeated oral dose (human) | No obvious toxic effects after ingesting 2000 mg/day for 30 days |
| Skin irritation (human) | 5% solution in Propylene glycol was non-irritant. (5 day covered contact in 50 human volunteers) |
| Eye irritation (human) | Solutions containing 0,1-0,3% methyl paraben produced symptoms of irritation and a burning sensation which disappeared within 1 minute |
| Skin sensitisation (human) | No evidence of skin sensitisation in healthy subjects |
| Photosensitisation (human) | No evidence of photosensitisation |
| Carcinogenicity | No evidence of carcinogenicity |
| Ames test | No evidence of mutagenicity |

### Ethyl paraben

| **TEST** | **RESULT** |
|---|---|
| Acute oral LD₅₀ (Rat) | >4300 mg/kg |
| Repeated oral dose (Rat) - 25 weeks | Diet containing 2% ethyl paraben gave no visible signs of toxicity. Reduced growth rate was observed |
| Skin irritation (human) | 7% solution in propylene glycol was non-irritant. (5 day covered contact in 50 human volunteers |
| Eye irritation (rabbit) | Neat ethyl paraben produced slight irritation |
| Skin sensitization (human) | No evidence of skin sensitisation in healthy subjects |
| Photosensitization (human) | No evidence of photosensitisation |
| Carcinogenicity | No evidence of carcinogenicity |
| Ames test | No evidence of mutagenicity |

### Propyl paraben

| **TEST** | **RESULT** |
|---|---|
| Acute oral LD₅₀ (Mouse) | >8000 mg/kg |
| Intraperitoneal L_{D50} (Mouse) | 640 mg/kg |
| Repeated oral dose (Rat) - 96 weeks | 900-1200 mg/kg/day gave no visible signs of toxicity or tissue changes in the major organs |
| Repeated oral dose (human) | No obvious toxic effects in 28 patients after ingesting 150 mg/day for 4-7 days |
| Skin irritation (human) | 12% solution in Propylene glycol was non-irritant. (5 day covered contact in 50 human volunteers) |
| Eye irritation (human) | A solution containing 0,01% propyl paraben produced no symptoms of irritation in a contact time of 15 minutes in 60 patients |
| Skin sensitisation (human) | No evidence of skin sensitisation inhealthy subjects |
| Photosensitisation (human) | No evidence of photosensitisation |
| Carcinogenicity | No evidence of carcinogenicity |
| Ames test | No evidence of mutagenicity |

### Propylene glycol

According to "chemical resistance chart, 1996 Nalgene Labware catalogue", concentrated propylene glycol gives little or no damage after 30 days of constant exposure on polycarbonate at 20°C, and some effect after 7 days of constant exposure at 50°C. According to the same chemical resistance chart, concentrated propylene glycol has no damaging effect on polyethylene (filter-material) after 30 days exposure at 20°C and 50°C. Thus, the preferred concentration of propylene glycol (2%) does not have any effect on rigid plastic.

The lowest total paraben concentration, at which precipitation at 4°C is obtained, is the antimicrobial composition Pres. (21). In order to obtain a safety margin in solubility of 50 % at 4°C the composition Pres. (18) is a preferred choice.

### EXAMPLE 5. WASHING-OUT PROPERTIES.

In a safety evaluation it assumed that the entire volume of a housing is filled with the aqueous antimicrobial preservation composition as the worst case.

A housing of about 180 ml was filled with a separation matrix of an active polysaccharide gel, to which the protein avidin had been immobilized, and the composition Pres. (18).

The housing was inserted in its device for extracorporeal circulation treatment and rinsed with 900 ml of a Ringer-Acetate solution (Pharmacia, Inc) before clinical use. The residual concentration of the components of Pres. (18) after the rinsing procedure was then analyzed. The maximum concentrations found were below the detection limit:

| | |
|---|---|
| Methyl paraben | <0.010 g•l⁻¹ |
| Ethyl paraben | <0.010 g•l⁻¹ |
| Propyl paraben | <0.010 g•l⁻¹ |
| Propylene glycol | 0.005 g•l⁻¹ |

Assuming a device volume of 210 ml, the residuals would amount to:

| | |
|---|---|
| Methyl paraben | <2.1 mg |
| Ethyl paraben | <2.1 mg |
| Propyl paraben | <2.1 mg |
| Propylene glycol | <1.1 mg |

These amounts correspond to the following maximal intravenous doses to a 50 kg person:

| | |
|---|---|
| Methyl paraben | 0.042 mg/kg |
| Ethyl paraben | <0.042 mg/kg |
| Propyl paraben | <0.042 mg/kg |
| Propylene glycol | <0.022 mg/kg |

The safety margin between acutely toxic doses of parabens and the residual amounts in the device is sufficiently large and is not regarded as harmful to the patient. Animal studies and the very long use and experience from pharmaceuticals, indicate that there is no significant risk for chronic toxic effects.

The safety margin for propylene glycol is also sufficient large for both acute and chronic toxicity. The maximum residual amount of propylene glycol from the device is much less than the administered dose of several pharmaceuticals. The clinical experience of administration of such small doses of propylene glycol indicates that the administration would not be harmful.

An additional advantage of using an antimicrobial preservation composition according to the invention in these applications is the specific ultraviolet light absorption at 256 nm (E^{1%}₂₅₆ = 9.5), which enables a continuous monitoring of the rinsing procedure.

## Claims

1. Use of an aqueous antimicrobial preservation composition for eliminating or reducing the microbial content of a microbially contaminated separation matrix, which is to be used in a housing of a separation device, wherein said composition comprises at least one alkyl paraben.

2. Use as in claim 1, wherein said at least one alkyl paraben is methyl paraben, ethyl paraben, propyl paraben, or butyl paraben.

3. Use as in claim 1 or 2, wherein the concentration of said at least one alkyl paraben decreases with increasing alkyl number.

4. Use as in claim 2 or 3, wherein the concentration of methyl paraben is between 0.5 and 2 g•l⁻¹.

5. Use as in claim 2 or 3, wherein the concentration of ethyl paraben is between 0.01 and 0.5 g•l⁻¹.

6. Use as in claim 2 or 3, wherein the concentration of propyl paraben is between 0.25 and 0.25 g•l ⁻¹.

7. Use as in claim 2 or 3, wherein the concentration of butyl paraben is at least 0.002 g•l⁻¹.

8. Use as in any of claims 1-7, wherein said aqueous antimicrobial preservation composition further comprises a solubility increasing agent at a concentration that is sufficient to maintain said at least one alkyl paraben in solution.

9. Use as in claim 8, wherein said solubility increasing agent is propylene glycol.

10. Use as in claim 9, wherein the concentration of said propylene glycol is not more than 20 g•l⁻¹.

11. A method of producing a separation matrix with eliminated or reduced microbial content, the method comprising the steps of
providing said separation matrix, microbially contaminated, in a housing or container;
adding an aqueous antimicrobial preservation composition, which comprises at least one alkyl paraben, to said separation matrix in said housing or container;
allowing said aqueous antimicrobial preservation composition to exert its effect in said housing or container until the number of colony forming units (CFU) per g preservative composition is sufficiently reduced; and
rinsing said aqueous antimicrobial preservation composition from said housing or container.

12. The method as in claim 11, wherein said at least one alkyl paraben is methyl paraben, ethyl paraben, propyl paraben, or butyl paraben.

13. The method as in claim 11 or 12, wherein the concentration said at least one alkyl paraben decreases with increasing alkyl number.

14. The method as in claim 12 or 13, wherein the concentration of methyl paraben is between 0.5 and 2 g•l⁻¹.

15. The method as in claim 12 or 13, wherein the concentration of ethyl paraben is between 0.01 and 0.5 g•l⁻¹.

16. The method as in claim 12 or 13, wherein the concentration of propyl paraben is between 0.25 and 0.25 g•l⁻¹.

17. The method as in claim 12 or 13, wherein the concentration of butyl paraben is at least 0.002 g•l⁻¹.

18. The method as in any of claims 11-17, wherein said aqueous antimicrobial preservation composition further comprises a solubility increasing agent at a concentration that is sufficient to maintain said at least one alkyl paraben in solution.

19. The method as in claim 18, wherein said solubility increasing agent is propylene glycol.

20. The method as in claim 19, wherein the concentration of said propylene glycol is not more than 20 g•l⁻¹.

21. The method as in any of claims 11-20, wherein said aqueous antimicrobial preservation composition is sterilized before it is added to said separation matrix.

22. The method as in claim 21, wherein said aqueous antimicrobial preservation composition is sterilized by means of steam or filter sterilization.

23. The method as in any of claims 11-22, wherein said aqueous antimicrobial preservation composition is allowed to exert its effect for at least 6 h.

24. The method as in any of claims 11-23, wherein said aqueous antimicrobial preservation composition is allowed to exert its effect until US and/or European pharmacopeia test protocol is fulfilled.

## Patentansprüche

1. Verwendung einer wäßrigen antibiotischen Konservierungsrezeptur für das Beseitigen oder das Verringern des Mikrobeninhalts einer mikrobiell kontaminierten Abscheidungsmatrix, die in einem Gehäuse einer Abscheidungsvorrichtung verwendet werden soll, wobei die Rezeptur mindestens ein Alkylparaben enthält.

2. Verwendung nach Anspruch 1, wobei das wenigstens eine Alkylparaben ein Methylparaben, Ethylparaben, Propylparaben oder Butylparaben ist.

3. Verwendung nach Anspruch 1 oder 2, wobei die Konzentration des wenigstens einen Alkylparaben sich bei Zunahme der Alkylzahl verringert.

4. Verwendung nach Anspruch 2 oder 3, wobei die Konzentration des Methylparabens zwischen 0,5 und 2 g/l beträgt.

5. Verwendung nach Anspruch 2 oder 3, wobei die Konzentration des Ethylparabens zwischen 0,01 und 0,5 g/l beträgt.

6. Verwendung nach Anspruch 2 oder 3, wobei die Konzentration des Propylparabens zwischen 0,25 und 0,25 g/l beträgt.

7. Verwendung nach Anspruch 2 oder 3, wobei die Konzentration des Butylparabens mindestens 0,002 g/l beträgt.

8. Verwendung nach einem der Ansprüche 1-7, wobei die wäßrige antibiotische Konservierungsrezeptur ein löslichkeitssteigerndes Mittel mit einer Konzentration enthält, die ausreicht, um wenigstens ein Alkylparaben in gelöster Form beizubehalten.

9. Verwendung nach Anspruch 8, wobei das löslichkeitssteigernde Mittel Propylenglykol ist.

10. Verwendung nach Anspruch 9, wobei die Konzentration des Propylenglykols nicht mehr als 20 g/l beträgt.

11. Verfahren zur Herstellung einer Abscheidungsmatrix mit beseitigtem oder verringertem Mikrobeninhalt, das die Schritte aufweist:
Bereitstellen der mikrobiell kontaminierten Abscheidungsmatrix in einem Gehäuse oder einem Behälter;
Hinzufügen einer wäßrigen antibiotischen Konservierungsrezeptur, die mindestens ein Alkylparaben enthält, in die im Gehäuse oder im Behälter befindlichen Abscheidungsmatrix;
Wirkenlassen der wäßrigen antibiotischen Konservierungsrezeptur in dem Gehäuse oder in dem Behälter, bis die Zahl kolonienbildender Einheiten (KBE) pro g der Konservierungsrezeptur ausreichend verringert ist;
und Ausspülen der wäßrigen antibiotischen Konservierungsrezeptur von dem Gehäuse oder Behälter.

12. Verfahren nach Anspruch 11, wobei das mindestens eine Alkylparaben ein Methylparaben, Ethylparaben, Propylparaben oder Butylparaben ist.

13. Verfahren nach Anspruch 11 oder 12, wobei die Konzentration des mindestens einen Alkylparabenen mit steigender Alkylzahl abnimmt.

14. Verfahren nach Anspruch 12 oder 13, wobei die Konzentration des Methylparabens zwischen 0,5 und 2 g/l beträgt.

15. Verfahren nach Anspruch 12 oder 13, wobei die Konzentration des Ethylparabens zwischen 0,01 und 0,5 g/l beträgt.

16. Verfahren nach Anspruch 12 oder 13, wobei die Konzentration des Propylparabens zwischen 0,25 und 0,25 g/l beträgt.

17. Verfahren nach Anspruch 12 oder 13, wobei die Konzentration des Butylparabens mindestens 0,002 g/l beträgt.

18. Verfahren nach einem der Ansprüche 11 - 17, wobei die wäßrige antibiotische Konservierungsrezeptur ferner ein löslichkeitssteigerndes Mittel mit einer Konzentration enthält, die ausreicht, um das mindestens eine Alkylparaben in gelöster Form beizubehalten.

19. Verfahren nach Anspruch 18, wobei das löslichkeitssteigernde Mittel Propylenglykol ist.

20. Verfahren nach Anspruch 19, wobei die Konzentration des Propylenglykol nicht mehr als 20 g/l beträgt.

21. Verfahren nach einem der Ansprüche 11 - 20, wobei die wäßrige antibiotische Konservierungsrezeptur sterilisiert wird, bevor die Abscheidungsmatrix hinzugefügt wird.

22. Verfahren nach Anspruch 21, wobei die wäßrige antibiotische Konservierungsrezeptur mittels Dampf- oder Filtersterilisation entkeimt wird.

23. Verfahren nach einem der Ansprüche 11 - 22, wobei die wäßrige antibiotische Konservierungsrezeptur ihren Effekt für mindestens 6 Stunden ausübt.

24. Verfahren nach einem der Ansprüche 11 - 23, wobei die wäßrige antibiotische Konservierungsrezeptur ihren Effekt so lange ausübt, bis das US- und/oder europäische Arzneibuchtestprotokoll erfüllt ist.

## Revendications

1. Utilisation d'une composition de conservation antimicrobienne aqueuse pour éliminer ou réduire la teneur antimicrobienne d'une matrice de séparation contaminée par des microbes, qui doit être utilisée dans un logement d'un dispositif de séparation, ladite composition comprenant au moins un alkylparabène.

2. Utilisation selon la revendication 1, dans laquelle au moins ledit alkylparabène est le méthylparabène, l'éthylparabène, le propylparabène ou le butylparabène.

3. Utilisation selon la revendication 1 ou 2, dans laquelle la concentration dudit au moins un alkylparabène diminue au fur et à mesure qu'augmente le nombre de groupes alkyle.

4. Utilisation selon la revendication 2 ou 3, dans laquelle la concentration de méthylparabène se situe entre 0,5 et 2 g.l⁻¹.

5. Utilisation selon la revendication 2 ou 3, dans laquelle la concentration d'éthylparabène se situe entre 0,01 et 0,5 g.l⁻¹.

6. Utilisation selon la revendication 2 ou 3, dans laquelle la concentration de propylparabène se situe entre 0,25 et 0,25 g.l⁻¹.

7. Utilisation selon la revendication 2 ou 3, dans laquelle la concentration de butylparabène est d'au moins 0,002 g.l⁻¹.

8. Utilisation selon l'une quelconque des revendications 1 à 7, dans laquelle ladite composition de conservation antimicrobienne aqueuse comprend en outre un agent augmentant la solubilité à une concentration qui est suffisante pour maintenir au moins ledit alkylparabène en solution.

9. Utilisation selon la revendication 8, dans laquelle ledit agent augmentant la solubilité est le propylène glycol.

10. Utilisation selon la revendication 9, dans laquelle la concentration dudit propylène glycol ne dépasse pas 20 g.l⁻¹.

11. Procédé de production d'une matrice de séparation présentant une absence de microbes ou une teneur réduite en microbes, le procédé comprenant les étapes consistant à
fournir ladite matrice de séparation, contaminée par des microbes, dans un logement ou un conteneur ;
ajouter une composition de conservation antimicrobienne aqueuse qui comprend au moins un alkylparabène, à ladite matrice de séparation dans ledit logement ou conteneur ;
laisser ladite composition de conservation antimicrobienne aqueuse exercer son effet dans ledit logement ou conteneur jusqu'à ce que le nombre d'unités formant colonie (UFC) par g de composition de conservation soit suffisamment réduit ; et
rincer ladite composition de conservation antimicrobienne aqueuse dudit logement ou conteneur.

12. Procédé selon la revendication 11, dans lequel au moins un alkylparabène est le méthylparabène, l'éthylparabène, le propylparabène ou le butylparabène.

13. Procédé selon la revendication 11 ou 12, dans lequel la concentration dudit au moins un alkylparabène diminue au fur et à mesure qu'augmente le nombre de groupes alkyle.

14. Procédé selon la revendication 12 ou 13, dans lequel la concentration de méthylparabène se situe entre 0,5 et 2 g.l⁻¹.

15. Procédé selon la revendication 12 ou 13, dans lequel la concentration de d'éthylparabène se situe entre 0,01 et 0,5 g.l⁻¹.

16. Procédé selon la revendication 12 ou 13, dans lequel la concentration de propylparabène se situe entre 0,25 et 0,25 g.l⁻¹.

17. Procédé selon la revendication 12 ou 13, dans lequel la concentration de butylparabène est d'au moins 0,002 g.l⁻¹.

18. Procédé selon l'une quelconque des revendications 11 à 17, dans lequel ladite composition de conservation antimicrobienne aqueuse comprend en outre un agent augmentant la solubilité à une concentration qui est suffisante pour maintenir au moins ledit alkylparabène en solution.

19. Procédé selon la revendication 18, dans laquelle ledit agent augmentant la solubilité est le propylène glycol.

20. Procédé selon la revendication 19, dans laquelle la concentration dudit propylène glycol ne dépasse pas 20 g.l⁻¹.

21. Procédé selon l'une quelconque des revendications 11 à 20, dans lequel ladite composition de conservation antimicrobienne aqueuse est stérilisée avant d'être ajoutée à ladite matrice de séparation.

22. Procédé selon la revendication 21, dans lequel ladite composition de conservation antimicrobienne aqueuse est stérilisée par stérilisation à la vapeur ou par stérilisation par filtrage.

23. Procédé selon l'une quelconque des revendications 11 à 22, dans lequel on laisse ladite composition de conservation antimicrobienne aqueuse exercer son effet pendant au moins 6 h.

24. Procédé selon l'une quelconque des revendications 11 à 23, dans lequel on laisse ladite composition de conservation antimicrobienne aqueuse exercer son effet jusqu'à ce que le protocole de test de la pharmacopée US et/ou européenne soit rempli.
